# EUROPÄISCHE PATENTANMELDUNG

(11) **EP 1 273 318 A2**
(43) Veröffentlichungstag der Anmeldung: **08.01.2003**
(21) Anmeldenummer: 02090215.1
(22) Anmeldetag: 14.06.2002
(51) Int. Cl.: A61N 1/05

(54) **Führungsdraht**

(30) Priorität: 02.07.2001 DE 10132330
(71) Anmelder: BIOTRONIK Mess- und Therapiegeräte GmbH & Co Ingenieurbüro Berlin, 12359 Berlin (DE)
(72) Erfinder: van Sloun, Math J.J., 6129 BK Berg aan de Maas (NL); Vos, Dick, 7721 JO Dalfsen (NL); de Cock, Dr.Cornelius Carolus, 2011 KL Haarlem (NL)
(74) Vertreter: Eisenführ, Speiser & Partner

(57) **Zusammenfassung**

Der Erfindung liegt der Gedanke zu Grunde, einen intravaskulär einführbaren Führungsdraht (1), der zur Implantation von Katheter in ein Herz verwendet wird, mit einer Elektrode (2, 5) zu versehen. Der Führungsdraht (1) wird dabei zunächst als temporäre Elektrode (2, 5) zum Stimulieren, zum Defibrillieren und/oder zum Abtasten des Herzens verwendet, um die optimale Implantationsposition für den einzuführenden Katheter zu bestimmen. Wenn diese optimale Position bestimmt ist, wird der Führungsdraht (1) an dieser Position belassen und der Katheter wird entlang des Führungsdrahtes (1) an die ermittelte Position eingeführt und implantiert. Sobald der Katheter implantiert ist, wird der Führungsdraht (1) wieder entfernt.

## Beschreibung

Die vorliegende Erfindung betrifft einen steuerbaren Führungsdraht, insbesondere zum Einsatz während einer Implantation von Kathetern in ein Herz, wobei der Führungsdraht dazu ausgebildet ist, intravaskulär eingeführt zu werden, so dass ein Katheter dann zur Implantation entlang des Führungsdrahtes einführbar ist, und wobei der Führungsdraht dazu ausgebildet ist, nach der Implantation des Katheters wieder entfernt zu werden.

Bei Patienten mit einem ausgeprägten Herzversagen kann sich ein Stimulieren des Herzens als vorteilhaft erweisen. Aber lediglich bei einer kleinen Gruppe (mit einem langen PR-Intervall) führte ein Stimulieren im rechten Ventrikel zu signifikanten Verbesserungen. Zur Modifikation der linken ventrikulären Funktion des Herzens wurden andere Stimulationsanordnungen untersucht. Es stellte sich heraus, dass eine optimale Stimulierung des Herzens hinsichtlich des hämodynamischen Zustandes des Herzens sich für jeden Patienten unterschiedlich darstellt.

Zur Ermittlung einer optimalen Stimulationsanordnung für den Patienten ist es somit unumgänglich, das Herz des Patienten zunächst hinsichtlich einer optimalen Therapie sowie einer optimalen Anordnung der jeweiligen Elektroden im Hinblick auf die gewünschten hämodynamischen Werte zu untersuchen.

Aus US 5,549,109 ist ein Katheter sowie ein Führungsdraht zum Abbilden der coronalen elektrischen Aktivität in den coronalen Arterien und/oder Venen bekannt, wobei sowohl Katheter als auch Führungsdraht Elektroden aufweisen. Der Führungsdraht und der Katheter kann von einer externen Stelle, wie beispielsweise die femoral Arterie oder Vene, in die cardialen Arterien oder Venen eingeführt werden. Dort werden der Führungsdraht und der Katheter derart platziert, dass die lokale cardiale elektrische Aktivität in der cardialen Muskelwand gemessen und überwacht wird. Durch die Variation der Messstellen in dem coronalen vaskulären System kann die elektrische Aktivität des Herzens komplett abgebildet werden.

Wenn die elektrische Aktivität des Herzens komplett abgebildet worden ist, wird der Katheter nebst Führungsdraht wieder aus den Patienten entfernt, damit eine elektrische Therapievorrichtung mit einem entsprechenden Katheter zum Stimulieren und/oder Defibrillieren des Herzens dauerhaft implantiert werden kann. Das Einführen des Katheters an die gewünschte Position erweist sich dabei als sehr komplex, da eine ungenaue Plazierung sich negativ auf die Stimulations- und Defribillationseigenschaften auswirkt. Es ist dabei vor allem sehr schwierig, die optimale Plazierung für den Katheter exakt wieder aufzufinden.

Aufgabe der Erfindung ist es somit einen Führungsdraht vorzusehen, mit dem die Plazierung eines einzuführenden Katheters verbessert werden kann.
Diese Aufgabe wird durch einen Führungsdraht der eingangs genannten Art mit den kennzeichnenden Merkmalen des beigefügten Anspruchs 1 gelöst.

Der Erfindung liegt dabei der Gedanke zu Grunde, einen intravaskulär einführbaren Führungsdraht, der zur Implantation von Katheter in ein Herz verwendet wird, mit einer Elektrode zu versehen. Der Führungsdraht wird dabei zunächst als temporäre Elektrode zum Stimulieren, zum Defibrillieren und/oder zum Abtasten des Herzens verwendet, um die optimale Implantationsposition für den einzuführenden Katheter zu bestimmen. Wenn diese optimale Position bestimmt ist, wird der Führungsdraht an dieser Position belassen und der Katheter wird entlang des Führungsdrahtes an die ermittelte Position eingeführt und implantiert. Sobald der Katheter implantiert ist, wird der Führungsdraht wieder entfernt.

Die mit der Erfindung einhergehenden Vorteile liegen insbesondere darin, dass die optimale Implantationsposition für den zu implantierenden Katheter durch einen Führungsdraht mit einer Elektrode ermittelt wird, der Führungsdraht an dieser Position belassen wird und der Katheter entlang des Führungsdrahtes zu dieser Position eingeführt wird. Damit wird sichergestellt, dass der Katheter an der exakten ermittelten Position implantiert wird, wodurch optimale Stimulations- und Defribillationseigenschaften erhalten werden.

Bei einer bevorzugten Ausgestaltung der Erfindung wird der Führungsdraht mit einer einzigen Elektrode versehen, welche sich vorzugsweise am distalen Ende des Führungsdrahtes befindet.

Bei einer weiteren Ausgestaltung der Erfindung werden Röntgenmarker an dem Führungsdraht angebracht. Diese Röntgenmarker dienen dazu, die ermittelte Implantationsposition für den zu implantierenden Katheter auch außerhalb des Körpers sichtbar zu machen, damit der die Implantation durchführende Arzt die Implantation überwachen kann und die ermittelte Implantationsposition leichter wieder auffindet.

Bei noch einer weiteren Ausgestaltung der Erfindung wird ein mechanischer oder ein magnetischer Anschlag am distalen Ende des Führungsdrahtes angebracht, damit dieser das Ende des Führungsdrahtes anzeigen kann und der die Implantierung durchführende Arzt den Katheter nicht über das distalen Ende des Führungsdrahtes hinausschiebt und somit die optimale Implantationsposition verfehlt.

Bei einer weiteren bevorzugten Ausgestaltung der Erfindung ist eine optische Markierung am proximalen Ende des Führungsdrahtes angebracht, damit erkennbar wird, wann der einzuführende Katheter bis an die ermittelte Implantationsposition vorgeschoben worden ist. Auch diese optische Markierung dient dazu, dass der Katheter nicht über das distalen Ende des Führungsdrahtes hinaus vorgeschoben wird und somit die optimale Implantationsposition verfehlt.

Bei einer weiteren besonders bevorzugten Ausgestaltung der Erfindung weist der Führungsdraht einen Durchmesser von 0,4 bis 0,8 mm auf. Durch diesen Durchmesserbereich wird sichergestellt, dass der Führungsdraht an alle gewünschten Positionen in dem cardiovaskulären System gelangen kann.

Weitere Ausgestaltungen der Erfindung sind Gegenstand der Unteransprüche.

Im Folgenden werden Ausführungsbeispiele der Erfindung anhand der Zeichnung näher erläutert. Dabei zeigen:
Fig. 1 eine Schnittansicht eines Führungsdrahtes, und
Fig. 2 einen Querschnitt am distalen Ende des Führungsdrahtes von Fig. 1.

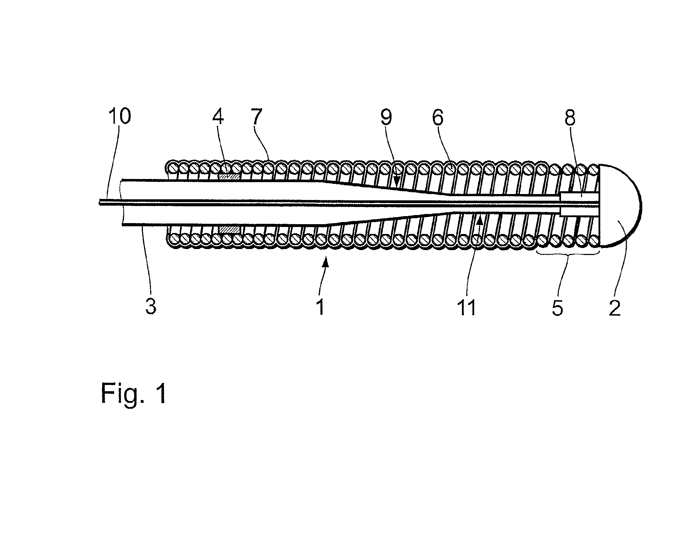

Fig. 1 zeigt eine Schnittansicht eines Führungsdrahtes 1. Der Führungsdraht 1 weist eine Helix-Wendel 6 auf, die bis zum dem distalen Ende eines Schaftes 3 reicht. Der Schaft 3 ist an seinem distalen Ende mit einer abgerundeten Spitze 2 und ferner über eine Lötstelle 4 mit der Wendel 6 verbunden.

Der drahtförmige, teflon-beschichtete Schaft 3 weist distal zu einem sich verjüngenden Bereich 9 einen Bereich 11 mit verminderter Breite und einen weiteren Bereich 8 am distalen Ende des Schaftes 3 auf, welcher flachgepresst ausgestaltet und mit der abgerundeten Spitze 2 verbunden ist. Durch das Flachpressen des Schaftes 3 an seinem distalen Ende, d. h. im Bereich 8, wird die Torsionsübertragung erhöht und gleichzeitig die Ermüdungsgefahr verringert.

Der Führungsdraht 1 weist im wesentlichen - mit der Ausnahme des Abschnittes 5 - an seiner Außenoberfläche eine Teflonbeschichtung 7 auf, welche als Isolierung verwendet wird. Diese Beschichtung dient außerdem der Verminderung der Reibung des Führungsdrahtes. Der nicht-isolierte Abschnitt 5 wird als Elektrode verwendet, deren elektrische Kontaktierung über die Wendel 6 erfolgt.

Die Windungen der Wendel 6 sind an ihrem distalen Ende, d.h. im Bereich des Abschnitts 5, leicht geöffnet, um die Flexibilität des distalen Endes des Führungsdrahtes 1 zu erhöhen.

Ferner weist der Führungsdraht 1 einen Steuer-Draht 10 auf, welcher an seinem distalen Ende an der abgerundeten Spitze 2 befestigt ist und sich bis zum proximalen Ende des Führungsdrahtes 1 erstreckt.

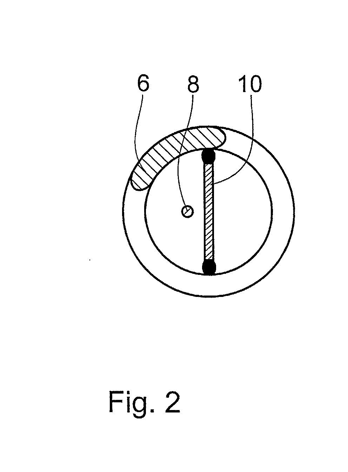

Fig. 2 zeigt einen Querschnitt des Führungsdrahtes 1 in seinem distalen Bereich. Das flachgepresste distale Ende 8 des Schaftes 3 und das distale Ende des Steuer--Drahtes 10 sind dabei in der abgerundeten Spitze 2 befestigt. Um den Schaft 3 sowie den Steuer-Draht 10 ist die Wendel 6 angeordnet.

Wenn der Steuer-Draht 10 in proximaler Richtung betätigt bzw. gezogen wird, erfährt das distale Ende des Führungsdrahtes 1 eine Verbiegung entsprechend dem Ausmaß der Bestätigung. Dieser Steuer-Draht 10 wird zum Manövieren in Arterien und Venen entsprechend betätigt, damit sich das distale Ende des Führungsdrahtes 1 verbiegt und der Führungsdraht so um eine Biegung oder in eine weitere Arterie oder Vene vorgeschoben werden kann. Da der Führungsdraht 1 mit Hilfe des Steuer-Drahtes 10 lediglich in eine Richtung verbogen werden kann, ist es notwendig, den gesamten Führungsdraht zu drehen, sobald die Verbiegung des distalen Ende des Führungsdrahtes 1 in eine andere Richtung erfolgen soll. Dies erfolgt durch Drehung des Schaftes 3, welcher mit der Wendel 6 über die Lötstelle 4 und mit der abgerundeten Spitze 2 verbunden ist.

Wie vorstehend beschriebenen wird der Führungsdraht 1 durch Arterien und Venen manövriert, um in das Atrium oder den Ventrikel zu gelangen. Wenn das distale Ende des Führungsdrahtes 1 die gewünschte Stelle im Atrium oder im Ventrikel erreicht hat, wird der nicht-isolierte Abschnitt 5 als monopolare Elektrode verwendet. Diese monopolare Elektrode 5 wird dabei als temporäre Elektrode verwendet, um das umliegende Gewebe abzutasten, zu stimulieren und/oder zu defibrillieren. Die Ergebnisse der Abfassung, der Stimulation und der Defibrillation an dieser Position werden erfasst. Diese Erfassung wird für mehrere Positionen im Atrium und im Ventrikel durchgeführt, um die optimale Position für einen zu implantierenden Katheter zum Abtasten, Stimulieren oder Defibrillieren zu finden.

Wenn die optimale Position gefunden ist, wird das distale Ende des Führungsdrahtes an dieser Position belassen und der einzuführende Katheter wird entlang des Führungsdrahtes 1 bis zum distalen Ende des Führungsdrahtes 1 eingeführt ("over the wire" oder "monorail"). Sobald der Katheter eingeführt und implantiert ist, wird der Führungsdraht 1 wieder entfernt.

Der Führungsdraht 1 dient somit lediglich dem Auffinden einer optimale Implantationsposition sowie dem Einführen eines zu implantierenden Katheters an die gefundene optimale Implantationsposition. Der Führungsdraht 1 ist nicht für eine dauerhafte Implantation vorgesehen.

Der Führungsdraht kann beispielsweise im Bereich des Coronarsinus positioniert werden, um das linke Ventrikel zu stimulieren.

Alternativ zu der Verwendung des nicht-isolierten Abschnittes 5 als monopolare Elektrode kann auch die abgerundete Spitze 2 des Führungsdrahtes 1 als Elektrode vorzugsweise als Spitzenelektrode verwendet werden. Dazu wird der Abschnitt 5 mit einer Isolierung versehen und die Isolierung um die abgerundete Spitze 2 des Führungsdrahtes 1 wird entfernt. Hierbei kann die abgerundete Spitze 2 über den Schaft 3 elektrisch kontaktiert werden.

Der Durchmesser des Führungsdrahtes 1 liegt in dem Durchmesserbereich von 0,4 bis 0,8 mm.

Am distalen Ende des Führungsdrahtes 1 wird ein Röntgenmarker vorzugsweise aus Gold vorgesehen. Alternativ dazu kann die abgerundete Spitze 2 aus Gold gefertigt sein und somit als Röntgenmarker dienen. Anhand eines derartigen Röntgenmarkers kann die durch den Führungsdraht 1 als temporäre Elektrode ermittelte optimale Implantationsposition sichtbar gemacht werden, um diese dann mit einem Elektroden-Katheter, welcher ebenfalls mit einem Röntgenmarker ausgestaltet ist, exakt anfahren zu können.

Alternativ dazu ist eine mechanische Markierung in Form eines Anschlags am distalen Ende des Führungsdrahtes 1 denkbar, welche dafür sorgt, dass der Katheter beim Einführen an die ermittelte optimale Implantationsposition nicht unbeabsichtigt über das distale Ende des Führungsdrahtes hinaus eingeführt wird und somit die optimale Implantationsposition verfehlt. Der Anschlag darf dabei aber nicht unüberwindbar ausgestaltet sein, da der Führungsdraht sonst nicht mehr nach der Implantation entfernbar wäre. Als Alternative zu dem mechanischen Anschlag wäre auch ein magnetisch spürbarer Anschlag denkbar. Ein derartiger magnetischer Anschlag könnte beispielsweise durch Verwendung von Magneten am distalen Ende des Führungsdrahtes sowie am distalen Ende des zu implantierenden Katheters realisiert werden.

Eine weitere Alternative zur Vermeidung, dass der Katheter über das distalen Ende des Führungsdrahtes 1 hinaus eingeführt wird, stellt eine optische Markierung des Führungsdrahtes und/oder des Katheters jeweils in ihrem proximalen Bereich, d. h. außerhalb des Körpers des Patienten, dar. Anhand einer derartigen optische Markierung ist es dem Bediender des Führungsdrahtes 1 und des Katheters möglich, zu erkennen, wann das distale Ende des einzuführenden Katheters an das distale Ende des Führungsdrahtes gelangt ist, so dass der einzuführende Katheter sich somit an der gewünschten Implantationsposition befindet und implantiert werden kann.

## Patentansprüche

1. Steuerbarer Führungsdraht (1) , insbesondere zum Einsatz während einer Implantation von Kathetern in ein Herz,
wobei der Führungsdraht (1) dazu ausgebildet ist, intravaskulär eingeführt zu werden, um anschließend einen Katheter zur Implantation entlang des Führungsdrahtes (1) einführen zu können, und
wobei der Führungsdraht (1) dazu ausgebildet ist, nach der Implantation des Katheters wieder entfernt zu werden,
**gekennzeichnet durch**
eine Elektrode (2, 5), die an dem Führungsdraht (5) befestigt und dazu ausgebildet ist, im eingeführten Zustand des Führungsdrahtes (1) angrenzendes Gewebe zu stimulieren, zu defibrillieren und/oder abzutasten, um eine optimale intravaskuläre Implantationsstelle für den zu implantierenden Katheter zu bestimmen,
wobei der Führungsdraht (1) ausgebildet ist, den zu implantierenden Katheter entlang des Führungsdrahtes (1) an die **durch** die Elektrode bestimmte Implantationsstelle einzuführen.

2. Führungsdraht nach Anspruch 1,
**gekennzeichnet durch** eine einzige Elektrode (2, 5).

3. Führungsdraht nach Anspruch 2,
**gekennzeichnet durch** eine Elektrode (2, 5) am distalen Ende des Führungsdrahtes.

4. Führungsdraht nach einem der vorherigen Ansprüche,
**gekennzeichnet durch** mindestens einen Röntgenmarker an dem Führungsdraht, der dazu ausgebildet ist, die ermittelte Implantationsstelle außerhalb des Körpers sichtbar zu machen.

5. Führungsdraht nach einem der vorherigen Ansprüche,
**gekennzeichnet durch** einen mechanischen Anschlag am distalen Ende des Führungsdrahtes (1), der dazu ausgebildet ist, das distale Ende des Führungsdrahtes (1) anzugeben.

6. Führungsdraht nach einem der vorherigen Ansprüche,
**gekennzeichnet durch** einen magnetischen, spürbaren Anschlag am distalen Ende des Führungsdrahtes (1), der dazu ausgebildet ist, das distale Ende des Führungsdrahtes (1) anzugeben.

7. Führungsdraht nach einem der vorherigen Ansprüche,
**gekennzeichnet durch** eine optische Markierung am proximalen extrakorporalen Ende des Führungsdrahtes (1), welche dazu ausgebildet ist, sichtbar zu machen, wenn ein einzuführender Katheter bis an die ermittelte Implantationstelle vorgeschoben worden ist.

8. Führungsdraht nach einem der vorherigen Ansprüche,
**dadurch gekennzeichnet, dass** der Führungsdraht (1) einen Durchmesser von 0,4 bis 0,8 mm aufweist.

9. Führungsdraht nach einem der vorherigen Ansprüche,
**dadurch gekennzeichnet, dass** die elektrische Zuleitung für die Elektrode (5) des Führungsdrahtes (1) über eine Drahtwendel (6) erfolgt.

10. Führungsdraht nach einem der Ansprüche 1 bis 8,
**dadurch gekennzeichnet, dass** die elektrische Zuleitung für die Elektrode (2) des Führungsdrahtes (1) über einen Schaft (3) erfolgt.
